# EUROPÄISCHE PATENTSCHRIFT

(11) **EP 1 092 403 B1**
(45) Veröffentlichungstag und Bekanntmachung des Hinweises auf die Patenterteilung: **30.10.2002**
(21) Anmeldenummer: 99120235.9
(22) Anmeldetag: 11.10.1999
(51) Int. Cl.: A61F 5/56

(54) **Antischnarcheinrichtung**
Anti-snore device
Dispositif anti-ronflement

(43) Veröffentlichungstag der Anmeldung: 18.04.2001
(73) Patentinhaber: Stockhausen, Rolf, Dr., 40210 Düsseldorf (DE)
(72) Erfinder: Stockhausen, Rolf, Dr., 40210 Düsseldorf (DE)
(74) Vertreter: Paul, Dieter-Alfred, Dipl.-Ing.

(56) Entgegenhaltungen:
- EP-A- 0 371 889
- EP-A- 0 815 813
- DE-A- 19 545 562
- US-A- 4 350 154
- US-A- 5 462 066

## Beschreibung

Die vorliegende Erfindung betrifft eine Antischnarcheinrichtung mit einer gebogenen, eine Konvex- und eine Konkavseite aufweisenden Abdeckplatte, welche in den Raum zwischen den Lippen und den Zähnen eines Menschen zwecks Abdeckung der Zahnlücken einsetzbar ist, wobei an der Konkavseite ein mandibular gerichteter Vorsprung sowie ein palatinal gerichteter Vorsprung ausgebildet sind, die im eingesetzten Zustand die Zähne von Ober- und Unterkiefer hintergreifen und wobei das Material der Antischnarcheinrichtung zumindest bereichsweise aus einem plastifizierbaren Material besteht, so daß die Abdeckplatte sowie die Vorsprünge durch Zungen- bzw. Fingerdruck innig gegen die Zahnflächen drückbar sind. Des weiteren betrifft die Erfindung ein Verfahren zum Modellieren einer solchen Antischnarcheinrichtung.

Das Schnarchen wird beim Menschen dadurch verursacht, daß bei Mundatmung durch den Atemluftstrom die Weichteile des Rachens, das Zäpfchen, das Gaumensegel und die hinteren Zungenteile in Schwingungen versetzt werden. Dies geschieht vornehmlich dann, wenn die oberen Atemwege verengt sind und hierdurch der Luftstrom beschleunigt wird.

Eine solche Verengung entsteht unter anderem durch das Absinken der Zunge in den Rachenraum. Es sind deshalb eine Reihe von Antischnarcheinrichtungen konzipiert worden, die es zum Ziele haben, die Zunge während des Schlafs an dieser Absinkbewegung zu hindern (DE-C-666 588) bzw. den Unterkiefer und damit die Zunge nach vorne zu verlagern, um auf diese Weise die Atemwege freizuhalten.

Aus der US-PS 5,462,066 ist beispielsweise eine Antischnarcheinrichtung der eingangs genannten Art bekannt, deren Ziel es ist, den Unterkiefer in einer vorgezogenen Position zu fixieren. Diese Antischnarcheinrichtung weist eine Abdeckplatte auf, welche in den Raum zwischen den Lippen und den Zähnen eines Menschen einsetzbar ist, wobei an der zum Mundinnenraum gerichteten Konkavseite der Abdeckplatte ein mandibulär sowie ein palatinal gerichteter Vorsprung vorgesehen sind, die im eingesetzten Zustand die Zähne von Ober- und Unterkiefer derart hintergreifen, daß der Unterkiefer in einer vorgezogenen Position fixiert wird. Die Herstellung dieser Antischnarcheinrichtung erfolgt, indem ein Rohling der Antischnarcheinrichtung, der aus einem thermoplastischen Material besteht, erwärmt und auf diese Weise plastifiziert wird. Nach dem Einsetzen des weichen Rohlings in den Mund werden die Abdeckplatte und die Vorsprünge bei vorgeschobenem Unterkiefer modelliert, um die Form des Rohlings an den Mund des Patienten anzupassen.

Bei einer weiteren Antischnarcheinrichtung dieser Art, die aus DE 197 06 204 A1 bekannt ist, wird zusätzlich vorgeschlagen, die Abdeckplatte so auszubilden, daß sie sowohl die Zahnlücken der Schneidezähne, als auch die Zahnlücken der Backenzähne abdeckt, so daß der Mundraum vollständig nach außen hin abgedichtet wird. Durch diese Ausbildungsform wird erreicht, daß es nicht mehr darauf ankommt, daß der Mundrand abdichtend auf der Konvexseite der Abdeckplatte aufliegt. Somit kann nach Einsetzen der Antischnarcheinrichtung durch ein Saugreflex ein hoher Unterdruck erzeugt werden, der die Zunge fest an der Innenseite der Zähne angesaugt hält. Dies wiederum hält die Atemwege im Rachenraum offen, so daß die Geschwindigkeit des Atemluftstroms niedrig bleibt und damit die Weichteile nicht zum Flattern angeregt werden. Der Unterdruck bleibt wegen der lückenlosen Abdichtung lange erhalten.

Die bekannten Antischnarcheinrichtungen haben sich durchaus bewährt. Die Bestrebungen gehen jedoch dahin die Antischnarcheinrichtung weiter zu verbessern und die Effektivität zu erhöhen.

Aufgabe der Erfindung ist es daher, eine Antischnarcheinrichtung der eingangs genannten Art zu schaffen, die ein Schnarchen wirksam verhindert. Des weiteren soll ein Verfahren zur Herstellung einer solchen Antischnarcheinrichtung angegeben werden.

Diese Aufgage ist erfindungsgemäß dadurch gelöst, daß die Abdeckplatte sowie die Vorsprünge so ausgebildet sind, daß sie sowohl die Zähne als auch die daran anschließenden Bereiche des Zahnfleisches ummanteln und aus einem Material bestehen, das im nicht plastifizierten Zustand eine Haftwirkung besitzt.

Der Erfindung liegt der Gedanke zugrunde, eine Antischnarcheinrichtung zu schaffen, die exakt an die Zähne und den Kiefer des Patienten angepaßt und anmodelliert ist. Durch diesen fast luftdichten Einschluß der Unter - und Oberkieferzähne in einen Materialblock wird ein unwillkürliches Auseinandergleiten der Kiefer und damit ein Zurückgleiten von Zunge und Unterkiefer verhindert. Auβerdem wird eine unwillkürliche Saugbewegung mit der Folge einer Vorwärtsfixierung der Zunge durch den Unterdruck hinter die Zähne ermöglicht.

Zur Herstellung einer solchen Antischnarcheinrichtung wird eine Form aus weichem Material in den Mund eingebracht, wo das Material dann durch Zungen- und Fingerdruck an die Zähne und den Kiefer angepaßt und anmodelliert wird, wodurch sich eine innige Ummantelung der oberen und unteren Zahnreihen sowohl außen als auch innen ergibt. Nach Verfestigung des Materials, das hierbei im wesentlich weder schrumpfen noch sich weiten sollte, sind die Zähne nur mit kräftiger, willkürlicher Kieferöffnung aus dieser exakten Negativform der Kiefer zu lösen.

In bevorzugter Weise erfolgt die Herstellung dieser Antischnarcheinrichtung, indem nach Einbringung der weichen Form der Antischnarcheinrichtung in den Mund mit mäßig vorgeschobenem Unterkiefer ein tiefer Einbiß in das weiche Material derart erfolgt, daß Schneide-, Eck- und eventuell auch Molarzähne tief im Material stecken, ohne hierbei das Material zu durchbeißen. Der Unterkiefer verbleibt dann in einer vorgeschobenen Stellung, und zwischen dem Material der Abdeckplatte und den Vorsprüngen bleibt eine stegartige Verbindung - die Beißleiste - mit einer Dicke von etwa 1 cm zwischen dem Material der Antischnarcheinrichtungsfront hinter den Zähnen bestehen. Die Beißleiste ist dabei entsprechend dem Zahnverlauf vorzugsweise als Viertelkreis ausgebildet, wobei die hiervon ausgehenden Vorsprünge einen Schild mit einer ungefähren Höhe von 2,5 cm bilden und damit der durchschnittlichen freien Höhe der Zahnrückseiten entsprechen. Die seitliche Ausdehnung des Schilds erstreckt sich etwa bis vor die Molarzähne. Die vordere Abdeckplatte ist vorzugsweise ca. 3 cm hoch und erstreckt sich seitlich bis ca. vor die vorderen Modalzähne.

Als Material für die Antischnarcheinrichtung wird vorzugsweise ein thermoplastisches Material verwendet, das in nicht plastifiziertem Zustand eine Haftwirkung besitzt. Beispielsweise kann die Antischnarcheinrichtung aus Thermolyn-Pedilom® oder aus Polypslint® bestehen. Alternativ kann ein Zweikomponentenmaterial verwendet werden, wobei das eine Material plastisch verformbar und durch Zugabe eines weiteren Materials in einen im wesentlichen starren Zustand bringbar ist.

Hinsichtlich weiterer vorteilhafter Ausgestaltungen der Erfindung wird auf die Unteransprüche sowie die nachfolgende Beschreibung eines Ausführungsbeispiels unter Bezugnahme auf die beiliegende Zeichnung verwiesen. In der Zeichnung zeigt:

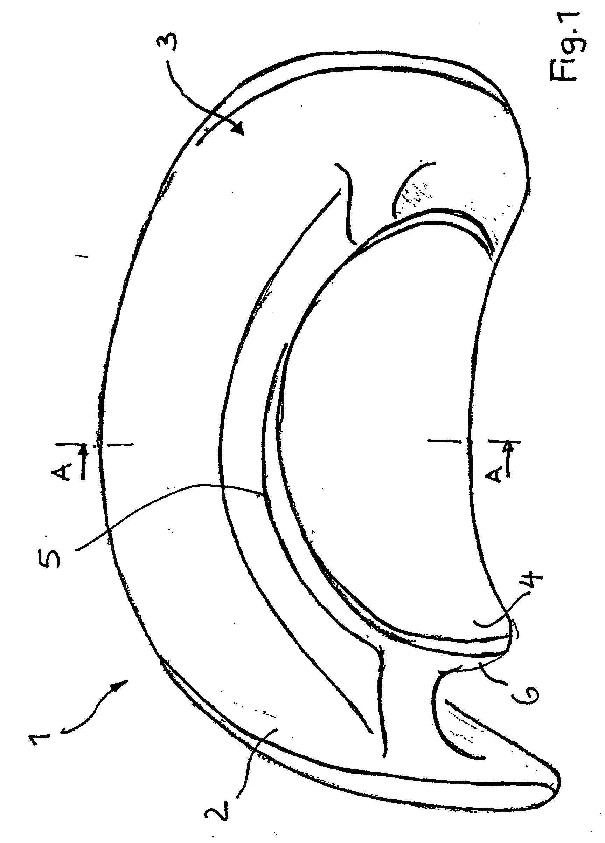

- Figur 1: eine Ausführungsform einer erfindungsgemäß Antischnarcheinrichtung in perspektivischer Ansicht,
- Figur 2: die Antischnarcheinrichtung aus Figur 1 im Schnitt entlang der Ebene A-A und
- Figur 3: die Antischnarcheinrichtung aus Figur 1 in Draufsicht.

Die in den Figuren dargestellte Antischnarcheinrichtung 1 besteht im wesentlichen aus einer Abdeckplatte 2, die derart bogenförmig gestaltet ist, daß sie nach dem Einsetzen in den Raum zwischen den Kiefern und den Lippen im wesentlichen alle Zähne, also auch die außenliegenden Backenzähne, abdeckt.

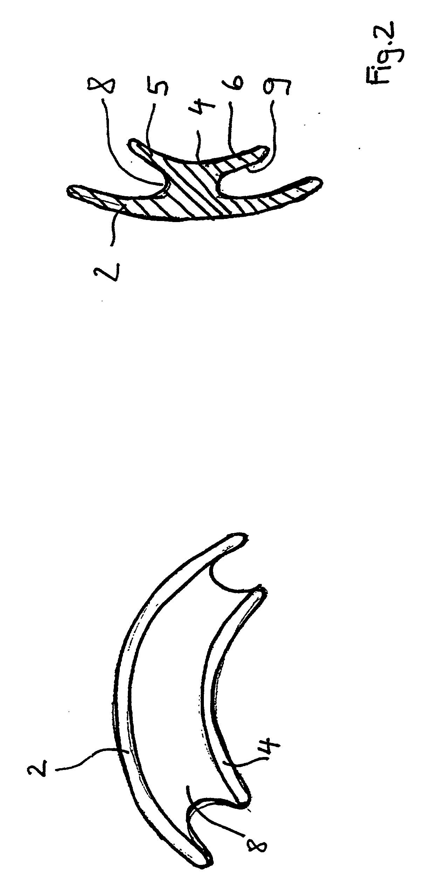

An der Konkavseite 3 der Abdeckplatte springt im Bereich der Mittelebene eine Beißleiste 4 vor. Wie insbesondere die Figuren 2 und 3 zeigen, ist die Beißleiste 4 entsprechend dem Zahnverlauf etwa als Viertelkreis ausgebildet und etwa 1 cm breit. An die Beißleiste 4 sind lingual und palatinal gerichtete Zahnhaltestege 5, 6 angeformt, die ein Schild 7 bilden, das eine Höhe von etwa 2,5 cm hat. Die Zahnhaltestege 5, 6 erstrecken sich im wesentlichen parallel zur Konkavseite 3 der Abdeckplatte 2, so daß Freiräume 8, 9 entstehen. In diese Freiräume 8, 9 fassen beim Einsetzen der Antischnarcheinrichtung 1 die obere bzw. die untere Zahnreihe ein. Wichtig ist hierbei, daß der linguale Zahnhaltesteg 6 die Zähne des Unterkiefers hintergreift und den Unterkiefer in einer gegenüber seiner Normalstellung vorgezogenen Stellung derart hält, daß die Schneidezähne von Ober- und Unterkiefer im wesentlichen einander gegenüberstehen. Durch diese Maßnahme wird erreicht, daß der Unterkiefer im Liegen in seiner vorgeschobenen Lage gehalten wird, wodurch die Atemwege freigehalten und somit ein Schnarchen verhindert werden kann.

Die Antischnarcheinrichtung 1 besteht durchgängig aus einem thermoplastischen Kunststoff mit niedriger Erweichungstemperatur, der bei Raumtemperatur im wesentlichen starr ist und eine Haftwirkung besitzt. Beispielsweise kann die Antischnarcheinrichtung 1 aus Thermolyn-Pedilom® oder Polysplint® bestehen. Durch Einlegung der Antischnarcheinrichtung 1 in heißes Wasser wird das Material plastisch verformbar. Nachdem die Außenfläche der Antischnarcheinrichtung 1 durch Eintauchen in kaltes Wasser abgekühlt worden ist, so daß sich der Benutzer nicht verbrennen kann, wird sie in den Mund eingesetzt. Dabei ist die Innentemperatur der Antischnarcheinrichtung 1 noch ausreichend hoch, so daß eine ausreichende Ummodellierung der Form möglich ist. Nach Einbringen der weichen Form in den Mund erfolgt ein tiefer Eindruck in das weiche Material derart, daß Schneide-, Eck- und eventuell auch Molarzähne tief in dem Material der Beißleiste 4 stecken bleiben, ohne hierbei das Material zu durchbeißen. Anschließend wird das ebenfalls weiche Material der Abdeckplatte 2 gegen die Vorderseite der Zähne und des Zahnfleisches gedrückt und an diesen anmodelliert, und wird in gleicher Weise das Material der Vorsprünge 5, 6 innenseitig mit der Zunge gegen die Zahnrückseiten sowie gegen das Zahnfleisch zu einer wenige Millimeter dicken, gebogenen Platte auseinandergewalzt. Bei diesem Modellierungsvorgang durch das Einbeißen und Andrücken ergibt sich eine innige Ummantelung der oberen und unteren Zahnreihen, und zwar sowohl innen als auch außen. Nach Erkalten des Materials, das hierbei im wesentlichen nicht schrumpfen und sich auch nicht weiten sollte, so daß das Material auch nach dem Erkalten noch an den Zähnen anliegt, sind die Zähne nur mit kräftiger, willkürlicher Kieferöffnung aus der exakten Negativform der Kiefer zu lösen. Dadurch, daß das Material selbst noch eine gewisse Haftwirkung besitzt, werden geringfügige Schrumpfmaße ausgeglichen.

Der fast luftdichte Einschluß der Unter- und Oberkieferzähne sowie des daran anschließenden Zahnfleisches in einem Materialblock verhindert ein unwillkürliches Auseinandergleiten der Zähne und damit ein Zurückgleiten von Zunge und Unterkiefer. Der Unterkiefer wird auf diese Weise unverrückbar auch bei relaxierten Muskeln in vorgeschobenem und geschlossenem Zustand fixiert.

Weiterhin ermöglicht es der fast luftdichte Einschluß, die Zungenspitze durch einen Saugreflex an den Zähnen bzw. an dem gebogenen Schild 7 zu fixieren, so daß sie entspannt im Mund liegt und von daher die Neigung des Benutzers, die Zunge unbewußt gegen die Saugwirkung zurückzuziehen, vermindert ist.

Ein Atemloch in der Antischnarcheinrichtung ist nicht notwendig. Überschüssiges Material kann beispielsweise durch Abschneiden mit einer Schere entfernt werden.

Zusammenfassend wird durch die erfindungsgemäße Ausbildung erreicht, daß auch durch wenig aufgetragenes Material vor den Zähnen ein Lippenschluß und hinter den Zähnen ein Saugreflex ermöglicht wird. Beide Eigenschaften halten die Atemluftströmung im Bereich des Rachens bis zum Kehlkopf frei von einengendem Weichteilgewebe, so daß ein Schnarchen sicher verhindert wird.

## Patentansprüche

1. Antischnarcheinrichtung (1) mit einer gebogenen, eine Konvex- und eine Konkavseite (3) aufweisenden Abdeckplatte (2), welche in den Raum zwischen den Lippen und den Zähnen eines Menschen zwecks Abdeckung der Zahnlücken einsetzbar ist, wobei an der Konkavseite (3) ein lingual gerichteter Vorsprung (6) sowie ein palatinal gerichteter Vorsprung (5) ausgebildet sind, die im eingesetzten Zustand die Zähne von Ober- und Unterkiefer hintergreifen und wobei das Material der Antischnarcheinrichtung zumindest bereichsweise aus einem plastifizierbaren Material besteht, so daß die Abdeckplatte (2) sowie die Vorsprünge (5, 6) durch Zungen- bzw. Fingerdruck innig gegen die Zahnflächen drückbar sind, wobei die Abdeckplatte (2) so ausgebildet ist, daß sie sowohl die Zähne als auch die daran auschließenden ßereiche des Zahnfleisches unmantelt, **dadurch gekennzeichnet, daß** die Vorsprünge (5, 6) so ausgebildet sind, daß sie sowohl die Zähne als auch die daran anschließenden Bereiche des Zahnfleischs ummanteln und die Abdeckplatte (2) sowie die Vorsprünge (5, 6) aus einem Material bestehen, das im nicht plastifizierten Zustand eine Haftwirkung besitzt.

2. Antischnarcheinrichtung nach Anspruch 1, **dadurch gekennzeichnet, daß** zumindest die Vorsprünge (5, 6) sowie die Abdeckplatte (2) aus einem durch Wärmeeinwirkung plastifizierbaren Material bestehen und so ausgebildet sind, daß sie im erwärmten Zustand durch Zungendruck verformbar und innig gegen die Zahnflächen sowie das Zahnfleisch drückbar sind.

3. Antischnarcheinrichtung nach Anspruch 1, **dadurch gekennzeichnet, daß** die Vorsprünge (5, 6) sowie die Abdeckplatte (2) aus einem Material bestehen, das durch Zugabe eines Härters aus einem plastisch verformbaren Zustand in einen im wesentlichen starren Zustand bringbar ist.

4. Antischnarcheinrichtung nach einem der vorherigen Ansprüche, **dadurch gekennzeichnet, daß** der linguale Vorsprung (6) derart ausgebildet ist, daß er im eingesetzten Zustand die Zähne des Unterkiefers derart hintergreift, daß der Unterkiefer in einer gegenüber seiner Normalstellung vorgezogenen Stellung gehalten wird.

5. Antischnarcheinrichtung nach einem der vorherigen Ansprüche, **dadurch gekennzeichnet, daß** die Vorsprünge (5, 6) ein bogenförmiges Schild (7) bilden, das mit der Abdeckplatte (2) durch eine Beißleiste (4) verbunden ist.

6. Antischnarcheinrichtung nach Anspruch 4, **dadurch gekennzeichnet, daß** die Beißleiste (4) eine Dicke von etwa 1 cm aufweist.

7. Antischnarcheinrichtung nach Anspruch 5 oder 6, **dadurch gekennzeichnet, daß** der Schild (7) eine Dikke von etwa 3 mm aufweist.

8. Antischnarcheinrichtung nach einem der Ansprüche 5 bis 7, **dadurch gekennzeichnet, daß** der Schild (7) eine Höhe von etwa 2,5 cm besitzt.

9. Antischnarcheinrichtung nach einem der vorherigen Ansprüche, **dadurch gekennzeichnet, daß** sie aus einem Zweikomponenten-Material besteht.

10. Verfahren zum Modellieren einer Antischnarcheinrichtung (1) mit einer gebogenen, eine Konvex- und eine Konkavseite (3) aufweisenden Abdeckplatte (2), welche in den Raum zwischen den Lippen und den Zähnen eines Menschen zwecks Abdeckung der Zahnlücken einsetzbar ist, wobei an der Konkavseite (3) ein lingual und ein palatinal gerichteter Vorsprung (5, 6) ausgebildet sind, die im eingesetzten Zustand die Zähne von Unterkiefer und Oberkiefer hintergreifen, wobei die Antischnarcheinrichtung in einem plastisch verformbaren Zustand in Ober- und Unterkiefer eingesetzt wird, die Abdeckplatte (2) sowie die Vorsprünge (5, 6) gegen die Zähne von Unter- und Oberkiefer gedrückt werden, und anschließend die Antischnarcheinrichtung (1) verfestigt wird, wobei die Abdeckplatte (2) so anmoduliert wird, daß die Zähne sowie die daran auschließenden Zahfleischbereiche unmantelt, **dadurch gekennzeichnet, daß** die Vorsprünge (5, 6) so anmoduliert werden, daß sie die Zähne sowie die daran anschließenden Zahnfleischbereiche ummanteln, und für die Antischnarcheinrichtung (1) ein Material verwendet wird, das im verfestigten Zustand eine Haftwirkung besitzt, so daß es an den Zähnen bzw. dem Zahnfleisch haftet.

11. Verfahren nach Anspruch 10, **dadurch gekennzeichnet, daß** für die Antischnarcheinrichtung ein thermoplastisches Material mit entsprechenden Haftungseigenschaften verwendet wird.

## Claims

1. An anti-snoring device (1) with a curved cover plate (2) having a convex and a concave side (3) which is insertable into the space between the lips and teeth of a person in order to cover the tooth spaces, there being configured on the concave side (3) a lingually directed projection (6) as well as a palatally directed projection (5) which, in the inserted state, engage behind the teeth of the upper and lower jaws, and the material of the anti-snoring device being made at least locally of a plasticizable material, so that the cover plate (2) as well as the projections (5, 6) can be pressed intimately against the tooth surfaces by tongue pressure or finger pressure, wherein the cover plate (2) is configured such that its envelops both the teeth and the regions of the gums adjacent thereto, **characterized in that** the projections (5, 6) are configured such that they envelop both the teeth and the regions of the gums adjacent thereto, and that the cover plate (2) and the projectionsare made of a material that possesses an adhesive effect in the non-plasticized state.

2. The anti-snoring device as defined in claim 1, **characterized in that** at least the projections (5, 6) as well as the cover plate (2) are made of a material that is plasticizable by the action of heat, and are configured such that in the heated state they are deformable by tongue pressure and can be pressed intimately against the tooth surfaces as well as the gums.

3. The anti-snoring device as defined in claim 1, **characterized in that** the projections (5, 6) as well as the cover plate (2) are made of a material that can be converted, by the addition of a hardener, from a plastically deformable state into a substantially rigid state.

4. The Anti-Snoring device as defined in one of the foregoing claims, **characterized in that** the lingual projection (6) is configured so that in the inserted state it engages behind the teeth in the lower jaw in such a way that the lower jaw is held in a position pulled forward with respect to its normal position.

5. The anti-snoring device as defined in one of the foregoing claims, **characterized in that** the projections (5,6) form an arc-shaped shield (7) that is joined to the cover plate by a bite strip (4).

6. The anti-snoring device as defined in claim 4, **characterized in that** the bite strip (4) has a thickness of approximately 1cm.

7. The anti-snoring device as defined in one of claim 5 or 6, **characterized in that** the shield (7) possesses a height of approximately 3 mm.

8. The anti-snoring device as defined in claims 5 through 7, **characterized in that** the shield (7) possesses a height of approximately 2.5 cm.

9. The anti-snoring device as defined in one of the forgoing claims, **characterized in that** it is made of a two-component material.

10. A method for molding an anti-snoring device (1) with a curved cover plate (2) having a convex and a concave side (3) which is insertable into the space between the lips and teeth of a person in order to cover the tooth spaces, there being configured on the concave side (3) a lingually and a palatally directed projection (5, 6) which, in the inserted state, engage behind the teeth of the upper and lower jaws, the anti-snoring device being inserted in a plastically deformable state into the upper and lower jaws, the cover plate (2) as well as the projections (5. 6) being pressed against the teeth of the lower and upper jaws and the anti-snoring device (1) then being solidified, wherein the cover plate (2) is molded on in such a way, that both the teeth and the regions of the gums adjacent thereto are envelopped, **characterized in that** the projections (5.6) are molded on in such a way that they envelop the teeth as well as the gum regions adjacent thereto, and what is used for the anti-snoring device (I) is a material that possesses an adhesive effect in the solidified state, so that it adheres to the teeth and gums.

11. The method as defined in claim 10, **characterized in that** a thermoplastic material with corresponding adhesion properties is used for the anti-snoring device.

## Revendications

1. Dispositif anti-ronflement (1) comportant une plaque de recouvrement courbe (2) présentant un côté convexe et un côté concave (3), qui peut être insérée dans l'espace situé entre les lèvres et les dents d'une personne afin de recouvrir les entredents, une saillie d'orientation linguale (6) et une saillie d'orientation palatine (5) étant formées sur le côté concave (3), qui à l'état inséré s'engagent derrière les dents de la mâchoire inférieure et de la mâchoire supérieure, et le matériau du dispositif anti-ronflement étant au moins pour partie un matériau plastifiable, de sorte que la plaque de recouvrement (2) ainsi que les saillies (5, 6) peuvent être intimement pressées contre les faces des dents en exerçant une pression avec la langue ou avec les doigts, la plaque de recouvrement (2) étant conçue de telle sorte qu'elle enveloppe à la fois les dents et les régions de la gencive qui se raccordent aux dents, **caractérisé en ce que** les saillies (5, 6) sont conçues de telle sorte qu'elles enveloppent à la fois les dents et les régions de la gencive qui se raccordent aux dents, et la plaque de recouvrement (2) ainsi que les saillies (5, 6) sont réalisées en un matériau qui possède un effet d'adhérence à l'état non plastifié.

2. Dispositif anti-ronflement selon la revendication 1, **caractérisé en ce qu'**au moins les saillies (5, 6) ainsi que la plaque de recouvrement (2) sont réalisées en un matériau plastifiable sous l'action de la chaleur, et sont conçues de telle sorte qu'à l'état échauffé, elles peuvent être déformées par la pression de la langue et pressées intimement contre les faces des dents ainsi que contre la gencive.

3. Dispositif anti-ronflement selon la revendication 1, **caractérisé en ce que** les saillies (5, 6) ainsi que la plaque de recouvrement (2) sont réalisées en un matériau qui, par addition d'un durcisseur, peut être transféré d'un état plastiquement déformable dans un état essentiellement rigide.

4. Dispositif anti-ronflement selon l'une quelconque des revendications précédentes, **caractérisé en ce que** la saillie linguale (6) est conçue de telle sorte qu'à l'état inséré, elle s'engage derrière les dents de la mâchoire inférieure de telle sorte que la mâchoire inférieure est maintenue dans une position avancée par rapport à sa position normale.

5. Dispositif anti-ronflement selon l'une quelconque des revendications précédentes, **caractérisé en ce que** les saillies (5, 6) forment un bouclier coudé (7), qui est relié à la plaque de recouvrement (2) par une bordure de dentition (4).

6. Dispositif anti-ronflement selon la revendication 5, **caractérisé en ce que** la bordure de dentition (4) présente une épaisseur d'environ 1 cm.

7. Dispositif anti-ronflement selon la revendication 5 ou 6, **caractérisé en ce que** le bouclier (7) présente une épaisseur d'environ 3 mm.

8. Dispositif anti-ronflement selon l'une quelconque des revendications 5 à 7, **caractérisé en ce que** le bouclier (7) possède une hauteur d'environ 2,5 cm.

9. Dispositif anti-ronflement selon l'une quelconque des revendications précédentes, **caractérisé en ce qu'**il est constitué d'un matériau à deux composants.

10. Procédé de modelage d'un dispositif anti-ronflement (1) comportant une plaque de recouvrement courbe (2) présentant un côté convexe et un côté concave (3), qui peut être insérée dans l'espace situé entre les lèvres et les dents d'une personne afin de recouvrir les entredents, une saillie d'orientation linguale (6) et une saillie d'orientation palatine (5) étant formées sur le côté concave (3), qui à l'état inséré s'engagent derrière les dents de la mâchoire inférieure et de la mâchoire supérieure, le dispositif anti-ronflement étant inséré dans un état plastiquement déformable dans la mâchoire supérieure et la mâchoire inférieure, la plaque de recouvrement (2) ainsi que les saillies (5, 6) étant pressées contre les dents de la mâchoire inférieure et de la mâchoire supérieure, puis le dispositif anti-ronflement (1) étant solidifié, la plaque de recouvrement (2) étant modelée de telle sorte qu'elle enveloppe les dents ainsi que les régions de la gencive qui se raccordent aux dents, **caractérisé en ce que** les saillies (5, 6) sont modelées de telle sorte qu'elles enveloppent les dents ainsi que les régions de la gencive qui se raccordent aux dents, et on utilise pour le dispositif anti-ronflement (1) un matériau qui possède un effet d'adhérence à l'état solidifié, de sorte qu'il adhère sur les dents et sur la gencive.

11. Procédé selon la revendication 10, **caractérisé en ce qu'**on utilise pour le dispositif anti-ronflement un matériau thermoplastique ayant des propriétés d'adhérence appropriées.
